# EUROPEAN PATENT APPLICATION

(11) **EP 0 887 403 A2**
(43) Date of publication of application: **30.12.1998**
(21) Application number: 98305060.0
(22) Date of filing: 26.06.1998
(51) Int. Cl.: C12N 1/20, C12N 1/21, C12R 1/63, C12N 1/36, C12N 15/74, A61K 39/106, A61K 39/10, C07K 14/245

(54) **Attenuated Vibrio cholerae strains**

(30) Priority: 27.06.1997 GB 9713664; 23.10.1997 GB 9722435
(71) Applicant: Chiron S.P.A., 53100 Siena (IT)
(72) Inventor: Fontana, Mariarita, 53100 Siena (IT); Pizza, Mariagrazina, 53100 Siena (IT); Rappuoli, Rino, 53010 Monteriggioni (SI) (IT)
(74) Representative: Hallybone, Huw George

(57) **Abstract**

The present invention relates to attenuated strains of *Vibrio cholerae* and their use as carrier agents for antigens in the mammalian body. The attenuated strains of the present invention can be used as carriers for both heterologous and homologous antigens. The strains of the present invention colonise the human intestine efficiently yet safely and generate antibodies with high bacteriocidal or anti-viral activity.

## Description

The present invention relates to attenuated strains of *Vibrio cholerae* and their use as carrier agents for antigens in the mammalian body. The attenuated strains of the present invention can be used as carriers for both heterologous or homologous antigens. The strains of the present invention colonise the human intestine efficiently yet safely and generate antibodies with high bacteriocidal or anti-viral activity.

Vaccination as a deliberate attempt to protect humans against disease has a long history. However, only in the latter half of the 20th century has technology allowed the development of sophisticated vaccination techniques. The advent of the new procedures of molecular genetics has greatly expanded the number of techniques that can be used for designing vaccines.

The general approach to vaccination has historically been to administer biological material from the organism responsible for the particular disease to a human subject. Such material was normally attenuated in some way so that it did not actually cause disease. The humoral response mounted by the immune system against the attenuated foreign proteins primed the body against a subsequent "real" infection, so reducing the incidence of serious disease.

Recent advances in genetic engineering have facilitated the generation of mutant proteins which can be screened for suitability as candidates for vaccines. In view of the potential significance of a successful vaccine, many groups have attempted to detoxify the pathogenic agents responsible for causing these diseases.

For example, in the case of enteropathogenic disease, attenuated non-toxic mutant enterotoxins have been developed that no longer induce pathogenic effects in the mammalian body, yet structurally are largely unmodified. Thus, on recognising these proteins as foreign bodies, the immune system directs specific antibodies to the attenuated toxins. These antibodies are then effective against wild type toxins on the occurrence of a genuine infection by live bacteria. Kaslow *et al.,* (1992) separately mutated Asp9 and His44 of cholerae toxin protein (CT) and truncated the protein after residue 180. These mutations attenuated the enzymatic activity of the toxin.

The elucidation of the three-dimensional structure of heat labile toxin protein (LT; Sixma *et al.,* 1991) allowed the rational mutagenesis of this protein and is likely to lead to the production of more effective toxoid molecules. Even before the structure was available, Tsuji *et al*.(1990) produced a non-toxic LT mutant that retained its immunogenicity by a single amino acid change. Harford *et al.,* (1989) described the production of a toxoid by *in vitro* mutagenesis of the LT-A gene from *E. coli* pathogenic for pigs.

Detoxified mutants of pertussis toxin have been reported to be useful both for direct intranasal vaccination and as a mucosal adjuvant for other vaccines (Roberts *et al.,* 1995). A known detoxifying mutation in the A subunit of *Bordetella pertussis* has been found to have an analogous effect in CT (Burnette *et al.,* 1991).

A number of attempts have utilised the ability to directly alter the function of bacterial or viral proteins so as to generate live strains that possess attenuated pathogenicity. Such "live" vaccines can only undergo limited replication in a host or encode detoxified proteins. Commonly, a broad spectrum of immunity can result following a single inoculation, and which can last for a lifetime. Such methods of vaccination have obvious advantages over multistage inoculation regimes using purified attenuated proteins that are expensive and usually require readministration in order to guarantee efficacy over long periods of time. In particular, the process of antigen purification for delivery to the body is expensive and in many cases may lead to denaturation of the protein.

There are also various problems associated with the use of attenuated "live" vaccines. Typically, these vaccines are viral-based and genetically plastic. Accordingly, a replicating virus may revert to a more pathogenic form and cause adverse reactions in a vaccinated patient or in a person having had contact with that patient.

Attenuated bacteria have recently been investigated as candidates for the delivery of antigens. A strain *of Salmonella typhi* (CVD908) appears to be a promising candidate for the delivery of antigens and has the added advantage that mucosal immunization with such strains elicits both mucosal and cell-mediated immune responses, in addition to stimulating serum antibodies.

Genetic manipulation of such strains should allow the concurrent administration of multiple antigens, so providing broad spectrum protection. Several groups have investigated this approach with the aim of generating a safe and effective multivalent oral diptheria-tetanus-pertussis (DTP) vaccine. To date, researchers have demonstrated the successful expression and protective ability of the non-toxic, immunogenic fragment C (FragC) of tetanus toxin in *S. typhimurium* (Chatfield *et al.,* 1992; Fairweather *et al,* 1990; Galen *et al.,* 1997).

The *Vibrio cholerae* bacterium has previously been investigated for suitability as a carrier for the Shiga toxin of the enteropathogens *E. coli* (Butterton *et al.,* 1995; Acheson *et al,* 1996) and *Shigella sonnei* (Viret *et al.,* 1996; Favre *et al.,* 1996).

The problem with these live oral vaccines is that they are often residually toxic for some individuals. Furthermore, there are presently no good vectors for the effective expression of cloned LPS-O antigen and CT-B genes in the human intestine.

Bacterial and viral-induced diseases therefore continue to be a major cause of suffering and death, particularly in the developing world. The poor health care in these areas and general poverty means that there is a desperate need for cheap, effective vaccines that do not need repeated administration in order to be continuously effective. Such vaccines would also significantly increase the efficacy of preventive medicine and reduce the costs of healthcare in the developed world.

It has been found that attenuated strains of *V. cholerae* are ideal candidates for use in the delivery of antigens for vaccination purposes. Infection with such agents is non-invasive, and ideal for vaccination purposes; this bacterium strikes the ideal balance between its ability to colonize the human intestine and therefore to invoke a mucosal immune response and its reactogenicity. These strains have been found to be effective carriers of both homologous and heterologous toxins and are therefore useful in the permanent vaccination of mammals against many forms of disease.

### Summary of the invention

According to the present invention, there are provided attenuated *Vibrio cholerae* strains that express one or more heterologous antigens or derivatives thereof, provided that the heterologous antigen is not derived from an enteropathogenic bacterium.

As used herein, the term "attenuated" means that the toxicity of the bacterial strain is diminished to negligible levels in mammals. Such strains are live, undergo a normal life cycle, yet secrete non-toxic mutant proteins or protein fragments. The host thus suffers none of the pathogenic symptoms that are associated with wild type (non-attenuated) strains, yet the immune system of the host is still exposed to the foreign proteins expressed by the bacterial agent.

It has been found that the bacterial strains of the present invention are particularly suitable for use as live vectors for the presentation of antigens in this context, due to their ability to successfully reach and colonise the small intestine in large numbers. The bacteria are able to penetrate the mucus layer and adhere to the mucosal epithelia, meaning that they successfully invoke an immune response.

These properties make attenuated *V. cholerae* an excellent choice as a vaccine delivery agent. Wild type *V. cholerae* is responsible for the widespread and contagious disease cholera, which causes severe intestinal disorders that prove fatal in a high percentage of recorded cases. Its pathological effects are exerted through the secretion of cholera toxin (CT) that consists of A and B subunits. The ADP-ribosylating activity of the A subunit contributes to intracellular toxicity in the host through stimulation of host adenylate cyclase, that results in severe dehydration of the body and loss of electrolytes. The B subunit is responsible for binding to eukaryotic cell surface receptors in the intestine.

The host for infection by the bacteria of the present invention is the human; humans are the only organisms that are susceptible to infection by *V. cholerae.*

By "heterologous antigen" is meant any protein or fragment that is not endogenous to *V. cholera.* The protein may be thus be derived from any source and may even be a protein endogenous to the host subject to be immunized, for example human tumour antigens or adhesins such as filamentary haemaglutinin (FHA), pertactin or fimbriae. Suitable proteins are immunogenic and thus invoke an immune response when expressed in an infected mammalian host. Most suitable, therefore, are proteins known to be particularly immunogenic.

Preferably, heterologous proteins produced according to the present invention are derived from known pathogens, such as viruses and bacteria.

In many cases, the heterologous protein may be harmful to the host if unmodified and therefore derivatives of the wild type protein should be used. As used herein, the term "derivatives" includes, for example, mutants containing amino acid substitutions, insertions or deletions that do not significantly alter the function or immunogenicity of the protein. In particular, conservative amino acid substitutions generally have little effect on the activity of protein molecules and are especially suitable.

Protein fragments are also suitable for use according to the present invention. The advantage of these molecules is that generally they exhibit significantly-reduced toxicity, yet retain their immunogenicity. Most suitable are fragments containing epitopes known to be most immunogenic, such as, for example, the fragment C of tetanus toxin from *Clostridium tetani.* The term "fragments" includes antigenic peptides that encompass or define antigenic epitopes.

The heterologous antigen expressed according to the present invention may be a toxin or toxin derivative. As used herein, the term "toxin" refers to any protein produced by a pathogen that is toxic to a host animal and may be synthetic, or naturally-derived, for example from bacteria or viruses. Preferably, toxins derived from bacterial pathogens are used in accordance with the present invention.

Toxins are particularly suitable for use according to the present invention, since any immune response invoked against the toxin will not only eradicate bacteria expressing toxins with recognised epitopes, but will also titrate out any copies of the toxin protein that are secreted from the bacteria before the bacteria themselves have been killed by the immune system.

Suitable toxins for use according to this aspect of the present invention include E6 and E7 from papilloma virus, gD2 from Herpes virus and gp120 from HIV. Other suitable toxins will be known to those of skill in the art.

According to a second aspect of the present invention, there is provided a bacterium of the IEM101 *V. cholerae* strain that expresses a heterologous or mutated homologous antigen or derivative thereof. This strain has been identified as a naturally-attenuated *V. cholerae* bacterium that we have shown to be safe in humans, which is able to colonise the human intestine and which is immunogenic.

As used herein, "mutated homologous antigen" refers to proteins endogenous to *V. cholerae* that have been modified from their wild type sequence in order to reduce their toxicity without significantly altering their structure. Their immunogenicity is thus unimpaired in the host animal.

Preferably, the mutated homologous antigen is a derivative of the CT protein.

The heterologous antigens or their derivatives suitable for use in the first aspect of the invention are also suitable for use in this aspect of the invention and include heat labile toxin protein from strains of *E. coli,* tetanus toxin, tracheal colonisation factor and pertussis toxin from *B. pertussis,* VacA, Nap and CagA from *H. pylori,* E6 and E7 from papilloma virus, gD2 from Herpes virus and gp120 from HIV. Other suitable toxins will be known to those of skill in the art.

It has been found, surprisingly, that in addition to its properties as a safe strain that is immunogenic and able to colonise the human intestine, the IEM101 strain is particularly useful to express certain proteins. For example, as described herein, expression of Nap in IEM101 leads to the formation of Nap decamers, as occurs in *H. pylori,* the organism in which this protein is naturally-produced. Furthermore, expression of CagA in the IEM101 strain gives efficient secretion of protein into the periplasm and into the culture supernatant, where the protein is expressed in its full length form. This strain thus has obvious advantages when used as an expression system.

It has also been found that expression of the mutated toxin proteins CTK63 and LTK63 leads to secretion of the polypeptides into the culture supernatant. It has further been found that the IEM101 strain is able to process the A subunit of LTK63 into the Al and A2 domains.

Toxin proteins of enterobacteria have been studied in great detail and it is known how to produce detoxified mutants of many known toxins (toxoids) from diverse pathogenic species. The term "toxoid" as used herein refers to a detoxified mutated toxin protein.

The term "detoxified" means that the mutant molecule exhibits a lowered toxicity relative to the wild type protein. Toxicity need not be completely abrogated, but must be sufficiently low to be used in an effective immunogenic composition without causing significant side-effects in the infected subject.

Toxicity may be measured using one of several methods. Such methods include testing toxicity in mouse cells, or CHO cells, or by evaluation of the morphological changes induced in Y1 cells. Preferably, toxicity is assessed by the rabbit ileal loop assay. Preferably, the toxicity of the toxoid is reduced 10,000-fold relative to its wild type counterpart as measured in Y1 cells or greater than 10-fold as measured by the ileal loop assay.

The now conventional techniques of recombinant DNA methodology can be used to introduce randomly or rationally-directed mutations into the amino acid sequence of the toxin molecule to produce toxoid molecules. Preferably, mutations are introduced by site-directed mutagenesis.

The encoding genes of many toxins suitable for use in this aspect of the present invention are known in the art. Of particular note is the definitive reference by Domenighini *et al.,* (1995) that reviews the sequences of toxin A and B subunits from various enterotoxic strains of *E. coli.*

The LT and CT toxins all comprise a single A subunit that is responsible for the enzymatic activity of the holotoxin (herein referred to as CT-A and LT-A) and five identical B subunits. These subunits are involved in binding to the intestinal epithelia.

Any alteration in amino acid sequence in any subunit, that has a detoxifying effect without significantly reducing immunogenicity is suitable for use according to the present invention. The immunogenic detoxified protein may adopt substantially the same structural conformation as the wild type naturally-occurring toxin. It is immunologically active and may cross-react with antibodies to the wild type toxins.

For example, with respect to CT-A and LT-A, any insertion, substitution or deletion mutation at, or in positions corresponding to Val-53, Ser-63, Val-97, Ala-72, Tyr-104 or Pro-106 will have the desired effect. Preferably, the mutation is a substitution for a different amino acid. For example, preferred substitutions include Val-53-Asp, Ser-63-Lys, Ala-72-Arg, Tyr-104-Lys or Pro-106-Ser.

Optionally, the CT or LT toxoid may contain other mutations such as, for example, substitutions at one or more of Arg-7, Asp-9, Arg-11, His44, Ser-61, His-70, His-107, Glu-110, Glu-112, Ser-114, Trp-127 or Arg-146. These mutations have been measured as being completely non-toxic or with a low residual toxicity as measured by the assays described above.

The amino acid residue or residues that replace the wild type in the protein sequence may be naturally-occurring or synthetic. Preferred substitutions are those that alter the amphotericity and/or hydrophilicity of the toxin whilst retaining as far as possible the structural integrity of the protein. Such amino acids have a similar steric effect to the wild type residue.

Preferably, with reference to CT, the mutation used is Ser-63-Lys (herein CTK63).

The heterologous or homologous antigens used in accordance with the present invention are preferably generated by recombinant means, by expression of the encoding gene or gene fragment in attenuated *V. cholerae* bacteria. Such expression methods are well known to those of skill in the art and many are described in detail by Sambrook *et al.* (1989).

Stable integration into the chromosome can be achieved most easily by homologous recombination, techniques of which are well known in the art and are described, for example in Sambrook *et al.* (1989).

Alternatively, the gene can be introduced into the bacterium in a plasmid, or in an expression vector. Introduction techniques are well known to those in the art and include transformation using CaCl₂, electroporation, conjugation or transduction.

Any suitable expression vector can be used for transformation of attenuated bacteria. The vector will contain a recombinant DNA molecule operatively linked to an expression control sequence, or a recombinant DNA cloning vehicle or vector containing such a recombinant DNA molecule under the control of a promoter recognised by the host transcription machinery. Preferably, a strong promoter is used to obtain high levels of expression of protein.

Preferably the gene is integrated into the bacterial chromosome.

Site-directed mutagenesis (SDM) is the method of preference used to generate mutated genes encoding antigens according to the present invention. There are many techniques of SDM now known to the man of skill in the art, including oligonucleotide-directed mutagenesis using PCR as set out, for example by Sambrook *et al.,* (1989) or using commercially available kits.

According to a further aspect of the present invention, there is provided an immunogenic composition comprising the attenuated strain of *V. cholerae* of the first or second aspect of the invention in conjunction with a pharmaceutically-acceptable carrier.

Pharmaceutically-acceptable carriers include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes) and inactive virus particles. Such carriers are well known to those of skill in the art. Additionally, these carriers may function as immunostimulating agents (adjuvants).

The composition may be used as a vaccine and may thus optionally comprise an adjuvant.

Suitable additional adjuvants to enhance effectiveness of the immunogenic compositions according to the present invention include, but are not limited to: (1) non-toxic mutants of heat labile toxin protein or pertussis toxin protein, such as described, for example in Tsuji *et al,* (1990); Harford *et al.,* (1989) and Roberts *et al.,* (1995); (2) oil-in-water emulsion formulations (with or without other specific immunostimulating agents such as muramyl peptides (see below) or bacterial cell wall components), such as for example (a) those formulations described in PCT Publ. No. WO 90/14837, including but not limited to MF59 (containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA)), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particles generated therefrom such as ISCOMs (immunostimulating complexes); (4) Complete Freunds Adjuvant (CFA) and Incomplete Freunds Adjuvant (IFA); (5) cytokines, such as interleukins (e.g., IL-1, IL-2, IL-4, IL-5, IL-6, IL-7, IL-12, etc.), interferons (e.g., gamma interferon), macrophage colony stimulating factor (M-CSF), tumor necrosis factor (TNF), etc; and (6) other substances that act as immunostimulating agents to enhance the effectiveness of the composition.

As mentioned above, muramyl peptides include, but are not limited to, N-acetyl-muramyl-L-threonyl-D-isoglutamine (thr-MDP), N-acetyl-normuramyl-l-alanyl-d-isoglutamine (nor-MDP), N-acetylmuramyl-1-alanyl-d-isoglutaminyl-1-alanine-2-(1'-2'-dipalmitoyl-*sn*-glycero-3-hydroxyphosphoryloxy)-ethylamine (MTP-PE), etc.

According to a further aspect of the invention, there is provided a process for the formulation of a vaccine composition comprising bringing attenuated bacteria according to the first or second aspect of the invention into association with a pharmaceutically-acceptable carrier, optionally with an adjuvant.

According to a still further aspect of the present invention, there is provided a method of vaccinating a mammal against a disease, comprising administering a preparation of bacteria according to the first or second aspect of the invention, that expresses an antigen associated with a pathogen responsible for the disease.

Bacteria of the genus *Vibrio cholerae ,* strain IEM101 are also provided as an emdoiment of the present invention. These bacteria possess the following properties:
biotype: T1 Tor; subbiotype 1a; serotype: Ogawa. The genotype can be classified as follows: Hly+; pili: TcpA+, Type B+, Type C-; ctx -; zot-; ace-; RS1+; st-; slt-; hly+;
tcpA+. Their biological properties can be assessed as follows: LD50#; Infant mouse test 1.58x10⁷; Adult mouse test 1.26x10⁷ (CFU); RIL test (ml/cm): Live cells (10¹²) 0; supernatant 0 (concentrated x50).

All documents mentioned in the text are incorporated herein by reference.

Various aspects and embodiments of the present invention will now be described in more detail by way of example. It will be appreciated that modification of detail may be made without departing from the scope of the invention.

### BRIEF DESCRIPTION OF THE FIGURES

Figure 1A: Levels of humoral anti-TT IgG were analyzed by ELISA.

Figure 1B shows the survival of immunised and non-immunised mice after challenge with 10 x LD₅₀ of tetanus toxin on day 70 after immunisation.

Figure 2 shows the bacterial number in tracheas and lungs from animals inoculated with IEM101[pNIR-tcf].

Figure 3: Expression of Nap in IEM101. Lanes 1 and 2, culture supernatant; lanes 3 and 4, 10ul of total cell extract; lane 5, purified Nap; lane 6 and 7, 30ul of total cell extract.

Figure 4: Localisation of Nap when expressed in IEM101. Lane 1, total cell extract; lanes 2 and 3, supernatant after sonication boiled and unboiled respectively; lane 4, pellet after sonication; lane 5, purified Nap; lane 6 total cell extract of bacteria grown in aerobic conditions; lane 7, total cell extract of bacteria grown in low aeration.

Figure 5: Western blot showing size separation of Nap when expressed in IEM101. Lane 1, purified Nap. Lane 2 to lane 7, fractions from gel filtration chromatography; lane 8, purified Nap, lane 9 starting material (soluble protein fraction).

Figure 6: Expression of CagA in IEM101. Lane 1, expression of CagA in *E. coli;* lane 2, soluble fraction from IEM101 expressing CagA; lane 3, periplasmic fraction from IEM101 expressing CagA; lane 4, supernatant of culture of IEM101 expressing CagA.

Figure 7: Western blot showing secretion of LTK63 and CTK63 into the supernatant of culture of IEM101 expressing these polypeptides. Lanes 1 and 4, purified cholerae toxin; lane 2, supernatant fraction from IEM101 expressing LTK63; lane 3, supernatant fraction from IEM101 expressing CTK63.

### EXPERIMENTAL

### Example 1: Isolation of attenuated live V. cholerae strains

Gao and co-workers have developed a phage-typing method by which *V. cholerae* 01 strains can be classified into subtypes. It was proposed that there are two types of *V. cholerae:* epidemigenic strains that are able to cause epidemics and pandemics (ESVC) and non-epidemigenic strains (NSVC), most of which do not cause diarrhoea and some of which can cause only mild diarrhoea (Oi and Gao, 1984).

In the present study, it was investigated whether some ESVC strains may have evolved to lose some of their pathogenic genes under natural environmental conditions, while retaining the unaltered cell envelope of the virulent strains. Such strains would be ideal as candidate vaccine strains for the delivery of antigens since it is thought that the main immunogens that confer immunity are often anti-bacterial rather than antitoxic.

The plasmids and strains used in the present study are shown in Table 1. The method of phage-sub-biotyping used was as set out in the Manual of prevention and treatment of cholerae (Ministry of Public Health of China, 1987). ³²P-labelled gene probes were used to detect the presence of *ctx, RS1, ace* and *zot* genes. Gene probes labelled with digoxin were used to detect *tcpA, sit* and *st* genes.

402 strains that were judged to be epidemigenic (as typed by phage-sub-biotyping) were examined by molecular genetic techniques. Fourteen ESVC isolates were identified that lacked the CT gene, 13 of which had RST flanking sequences.

Experiments were then carried out to assess the potential efficacy of these strains as vaccine candidates. In preliminary tests in rabbit ileal loop (RIL) assays, one of the 14 strains (designated IEM101) produced better protection than the others against challenge with virulent strains of *V. cholerae 01.* Rabbit ileal loop assays were carried out conventionally as described, for example in Guancai *et al.,* (1993). Briefly, rabbits hungered for one day were operated. The bowels were ligated into loops of about 10cm in length, then 1.0 ml bacterial liquid sample (containing varying amounts of bacteria) was added to each loop.

The studies on the selected molecular and biological characteristics of IEM101, and the results of the pathogenicity tests are summarised in Table 2. These characteristics give the strain the properties that make it ideal for use according to the present invention. To measure the LD₅₀ values of different ESVCs, the mouse and infant mouse tests were used, as described by Noeh and Rowly, (1972).

To ascertain the active protection of this strain in the RIL model, rabbits were first immunised as follows. Three groups of rabbits were immunised with strain IEM101 (Guancai *et al.,* 1993). In each group, animals received a single dose of either 10⁶, 10⁷ or 10⁸ live bacteria. Animals underwent laparotomy and organisms were instilled directly into the proximal small bowel. 14 days after immunisation, rabbits were challenged with either 10⁵, 10⁶ or 10⁷ live bacteria. The test was read at 20 hours and the results recorded as ml fluid/cm of intestine. All three protective doses of IEM101 were protective against the challenge strains (see Table 3).

Another group of animals were immunised with a high dose (10¹¹ IEM101 bacteria) and were bled after 28 days. The sera were used in passive experiments as follows: suspensions of organisms were mixed with sera at varying dilutions and then used in RILs or infant mouse tests to measure LD₅₀ values. The results are shown in Table 4.

Human volunteer studies (Wang *et al.,* 1992) were also conducted. 31 adult volunteers were divided into 7 groups. Each group was orally immunised with a single dose of either 10⁹ killed bacteria or 10², 10⁴, 10⁶, 10⁸, 10⁹, or 10¹⁰ live IEM101 bacteria. Organisms were given in 2% NaHCO₃ to protect against stomach acidity.

Stool samples were cultured for a period of two weeks. Serological analyses were carried out at 0, 7, 14, 28, 90, 180, 270 and 360 days respectively after immunisation. Vibriocidal antibody, agglutinative antibody and immunoglobulin isotyping (IgA, IgG and IgM) were carried out using standard methodologies and specific peripheral blood lymphocytes (PBL) assays according to standard techniques. The results of the colonisation and safety studies are shown in Tables 5 and 6. Immune responses are shown in Tables 7, 8, 9 and 10.

These results clearly show that the group immunised by 10⁹ live IEM101 is most efficacious. Furthermore, high levels of vibriocidal antibodies are seen, a phenomenon regarded to be the best predictor of protective immunity. The results of all these studies also indicate that the IEM101 strain colonises human intestine well.

### Example 2: Attenuated V. cholerae strain as a carrier for heterologous antigens

The genes encoding the tracheal colonisation factor (tcf) and fragment C of the tetanus toxin (TetC) were cloned under the control of various promoters. The expression of these proteins in the IEM101 strain was confirmed by Western blot analysis. IEM101 strains expressing these proteins were then used to immunise mice and their protective effect was evaluated against subsequent challenge from virulent strains. These strains have been deposited at the ATCC in accordance with the provisions of the Budapest Treaty and have been accorded the designations CMCC# 4731 (IEM101:CTK63), CMCC# 4732 (IEM101:TetC) and CMCC# 4734 (IEM101:tcf) at Chiron and ATCC 202029 (IEM101:CTK63), ATCC 98548 (IEM101:TetC) and ATCC 98547 (IEM101:tcf) at the American Type Culture Collection.

The TetC protein was expressed under the control of the pNirB promoter. The plasmid pTETnir15 (Barry *et al.,* 1996) contains the coding sequence of TetC, under the control of the *E. coli* modified *nir*B promoter, which is inducible under anaerobic conditions. This plasmid was introduced into *V. cholerae* IEM101 by electroporation; transformants were able to produce TetC, and expression was induced when bacteria were grown without aeration (in completely filled, tight capped 50ml Falcon tubes), showing that the *nirB* promoter is function in IEM101. The level of expression was estimated by immunoblotting to be 0.1ug/5x10⁸ cells. In the case of tcf, the results show that the highest level of expression was observed with the pNirB promoter and that the tcf was processed by IEM101 in the same way as by *B. pertussis* and was exposed on the mucosal surface.

The *tcf gene* was amplified from *Bordetella pertussis* ATCC BP18323 chromosomal DNA, using the two oligonucleotides:
Tcf1: 5'-ACTAGTGATCATATGCACAATTTACGGAAATA-3' which contains the initial codon of *tcf*, comprised in a *Nde*I site, and an upstream *Bcl*I site;
and Tcf2: 5'-CTCTAGAATTCTACCAGGCGTAGCGATACC-3', containing the stop codon of the *tcf gene* and an *Eco*RI site downstream.

The amplified *tcf* gene was cloned under the control of pT7, pCT and pNirB promoters. In the case of pT7, the amplification product was digested with *Bcl*I and *Eco*RI and cloned into pBLUESCRIPT/KS+ (Stratagene) between the *Eco*RI and *Bam*HI sites, originating the plasmid pT7Tcf

In the case of the pCT promoter, a 190bp sequence located immediately upstream to the ctx structural region was amplified from plasmid pJM17. Oligonucleotides Pct1:
5'-TAGATCTAGATACCTTTGCAGCGCAAGG-3' and Pct2:
5'-TATCTTTACCATATGATGCTCCC-3' were used respectively as forward and reverse primers; Pct1 contains an *Xba*I site at its 5' end, whereas Pct2, contains the initiation codon of *ctx* (reverse) inside a *Nde*I site. The amplified fragment was digested with *Xba*I and *Nde*I and cloned into pI7-tcf digested with the same enzymes, originating the plasmid pct-tcf.

In the case of the pNir promoter, the plasmid pTETnir15 was digested with *Bg*/II and *Bam*HI, liberating a fragment containing the coding region of TetC and about 30bp of the 5'-untranslated region, including the Shine-Delgarno sequence.

In order to reconstitute this 30bp 5' untranslated region, (which could be important for the RNA stability and/or efficient translation initiation) the plasmid pTETnir15 digested with *Bg*/II*-Bam*HI was ligated to a pair of annealed complementary oligonucleotides PnirUTR1:
5'-GATCTTAATCATCCACAGGAGACTTTCATATGATATCTAGATGCATC-3' and PnirUTR2:
5'-GATCGATGCATCTAGATATCATATGAAAGTCTCCTGTGGATGATTAA-3'
containing the 30bp 5' untranslated sequence, an *NdeI* site containing the ATG initiation codon, and other restriction sites *(Eco*RV, *Xba*I, *Nsi*I and *Cla*I) immediately downstream. This plasmid was called pnir100; it was digested with *Xba*I, filled-in with Klenow fragment, digested with *Nde*I and ligated to the *Nde*I*-Eco*RV fragment from pT7-tcf containing the *tcf* gene, originating the plasmid pnir-tcf.

IEM101 containing the recombinant genes expressing the TetC or the tcf antigens were used to immunise mice intranasally. The rationale to use the intranasal route was based on the evidence that the induction of an immune response in intranasally-immunised mice is much more efficient than in orally immunised mice.

For TetC, immunisations were carried out as follows. BALB/c mice without anaesthesia received four doses of about 5 x 10⁸ CFU of either IEM101 or IEM101[pTETnir15], grown without aeration, by the intranasal route. All animals presented vibriocidal activity in their sera, as determined using the following protocol. Vibriocidal activity assays were performed by incubating 10¹⁰ CFU in PBS with 20% rabbit complement and serial dilutions of the immunized animal sera in 100µl in 96-well tissue culture plates for 1 hour at 37°C; 100µl of BHI (brain and heart infusion, DIFCO) was then added to each well, and plates were further incubated for 1 hour at 37°C; absorbance at 570nm was then measured. Titers were calculated as the dilution of the serum that gave 50% growth inhibition, as compared to pre-immune sera diluted 1:25.

Levels of humoral anti-TT IgG were analyzed by ELISA. Anti-TT antibodies were analyzed by ELISA, using 200ng of formalin inactivated TT as coating antigen in 100µl PBS/well overnight at 40°C; after 3 washes with PBS-0.05% Tween 20 (PBS-T), plates were blocked with 200µl/well 1%BSA in PBS-T for 2 hours at 37°C, washed 3 times with PBS-T, incubated with serial dilutions of sera from immunized animals in 0.5% BSA in PBS-T, for 1.5 hour at 37°C; following 3 washes with PBS-T, plates were incubated with 0.5% BSA in PBS-T containing anti-mouse IgG antibody conjugated with HRP for 1.5 hour at 37°C, washed again 3 times with PBS-T and developed with p-nitrophenol (Sigma), and reading of the absorbance at 405nm were performed. Titers were calculated as the dilution that gave three times the reading of pre-immune sera diluted 1:100.

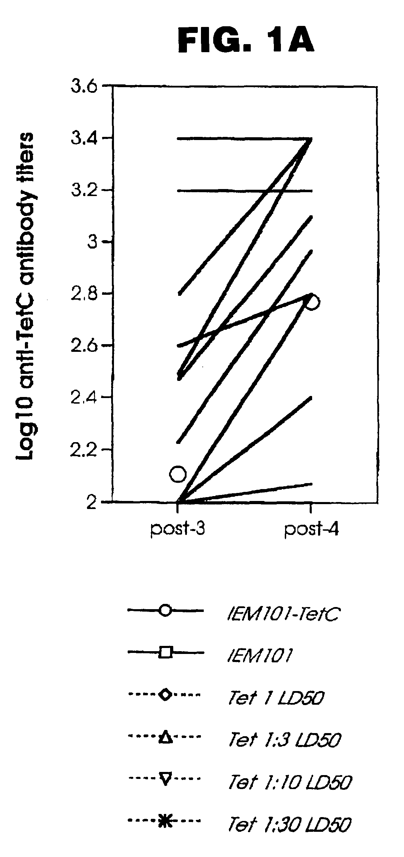

The results after three or four immunisations are shown in Figure 1A. Anti-TT IgA could also be detected in nasal washes, indicating that stimulation of local responses occurred (data not shown). Administration of heat inactivated IEM101[pTETnir15] did not induce detectable anti-TT antibodies.

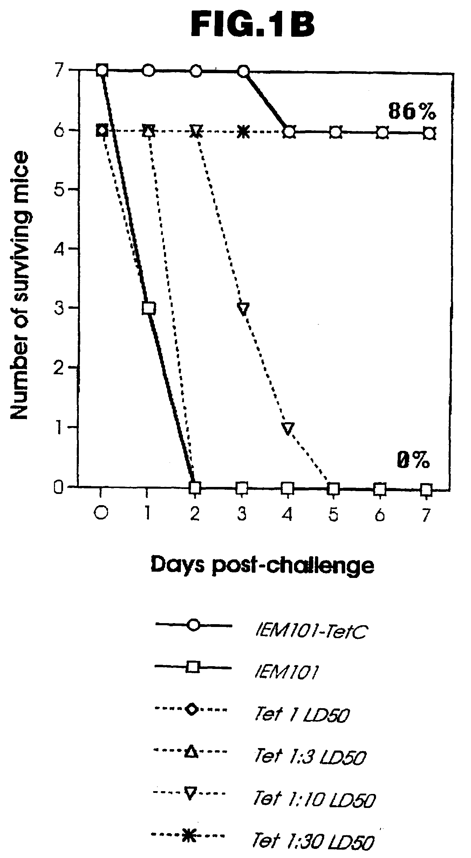

Animals were challenged with 10 x LD₅₀ of tetanus toxin on day 70. Six of seven animals that had received IEM101[pTETnir15] survived, whereas control animals (IEM101) died within 48 hours, demonstrating that the immune response raised by *Vibrio*-delivered TetC was able to neutralise the toxin and protect the animals (Figure 1B).

For immunisation with tcf, a similar procedure was carried out. 6-8 week old female BALB/c mice were immunized intranasally under light anaesthesia on days 0, 35 with about 5 x 10⁷ CFU of IEM101 and IEM101(pnir-tcf), grown under microaerobic condition for 4 hours at 37°C. On day 70, intranasal challenge with BP18323 Sm^{r} was performed and bacterial colonisation in trachea and lungs was followed.

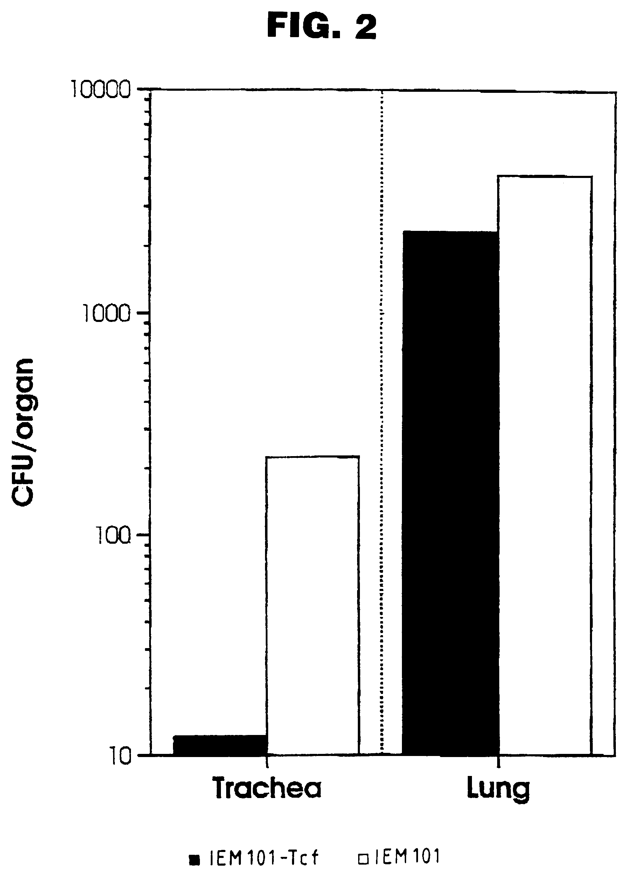

A significant decrease in bacterial number in tracheas from animals inoculated with IEM101[pNIR-tcf] could be observed on day 14 (shown in figure 2) as compared to the control group (inoculated with IEM101). No differences in Bordetella CFU numbers were detected at the lung level. (Figure 2).

Following intranasal challenge with a virulent *B. pertussis* strain there was a 10-fold reduction in the number of bacteria recovered by the trachea of the immunised mice with the IEM101 expressing tcf, compared to the mice immunized with IEM101, showing that, as for TetC, the response induced by tcf expressed *in V*. *cholerae* was protective.

### 2.1 NAP expression in IEM101

The plasmid pGEMnir-NAP has been obtained by cloning the gene coding for NAP under the control of the *nirB* promoter into the pGEM vector. The plasmid was introduced into IEM101 by electroporation; the recombinant strain has been named IEMnirNAP.

Two recombinant colonies were grown for 16 hours in LB + 100ug/ml of Ampicillin. Two milliliters of each culture were centrifuged, and the supernatants stored at 4°C until the analysis was performed. The pellet containing the bacterial cells was resuspended in 196µl of PBS. Culture supernatant and bacterial cells were then analysed for the presence of Nap protein by western blotting using rabbit anti-NAP polyclonal antibody at a dilution of 1:1000.

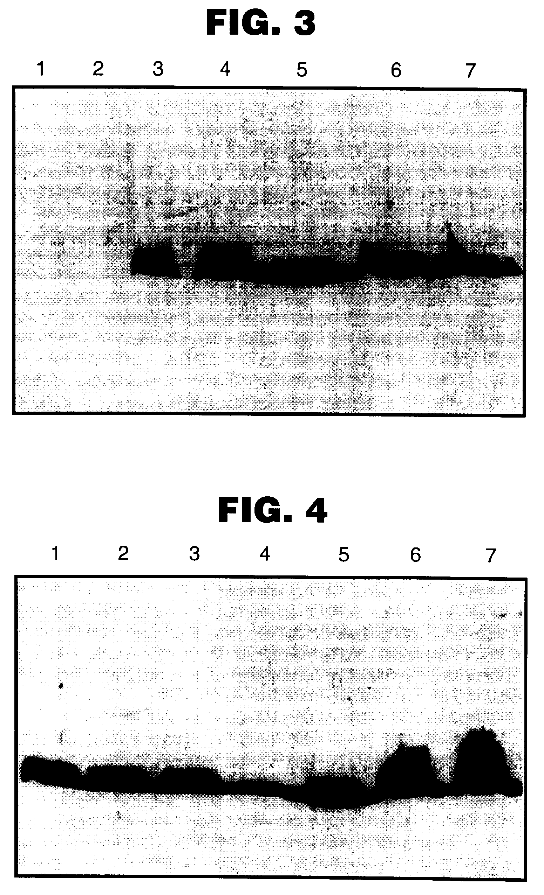

As shown in Figure 3 the protein is present in detectable amounts in the total extract when either 10ul (lanes 3 and 4) or 30ul (lanes 7 and 8) of both cultures were loaded on the gel, whereas the protein was absent in culture supernatants (lanes 1 and 20. These results show that IEM101 is able to produce the Nap protein and that the protein remains localised inside the cells and it is not secreted.

### 2.2 Nap localisation in IEM101.

1ml of an overnight culture of IEMnirNap was centrifuged and the pellet was resuspended in 500µl of PBS. 20µl of this bacterial suspension was stored for gel analysis, while 480ul was sonicated for bacterial disruption. After sonication the sample was centrifuged. The supernatant, containing the soluble fraction, was stored at 4 degrees, while the pellet was washed with 1 ml PBS and resuspended in 500µl of PBS. 20µl of total cell lysate, 20ul of the soluble fraction and 20ul of pellet after sonication, were loaded on a SDS gel and analysed by western blotting using the anti-Nap rabbit polyclonal antibody.

As shown in Figure 4, the Nap protein is present in the same amount in total cell lysate (lane 1), and in the soluble fraction (lanes 2 and 3) whereas is present in small amount in the pellet after sonication (lane 4). These results show that Nap is produced in IEM101 in a soluble form.

### 2.3 Induction of Nap expression

1ml of an overnight culture of IEMnirNAP strain was diluted 1:25 inLB medium. 15ml was grown at 37°C for 4.5 hours in a tight capped falcon tube (in low aeration) and 9 ml was grown at 37 degrees with aeration until the culture reached the OD₆₀₀ₙₘ of 0.5.

2ml of each culture was centrifuged and pellet containing bacterial cells was resuspended in PBS to obtain an OD₆₀₀ₙₘ of 0.5/ml. 30µl of each sample were loaded on a SDS gel and analysed by western blot for Nap production. The results reported in Figure 4 show that the amount of Nap produced is higher when the strain is grown under low aeration (lane 7), suggesting the the nirB promoter is induced.

### 2.4 Nap polymerization in IEM101.

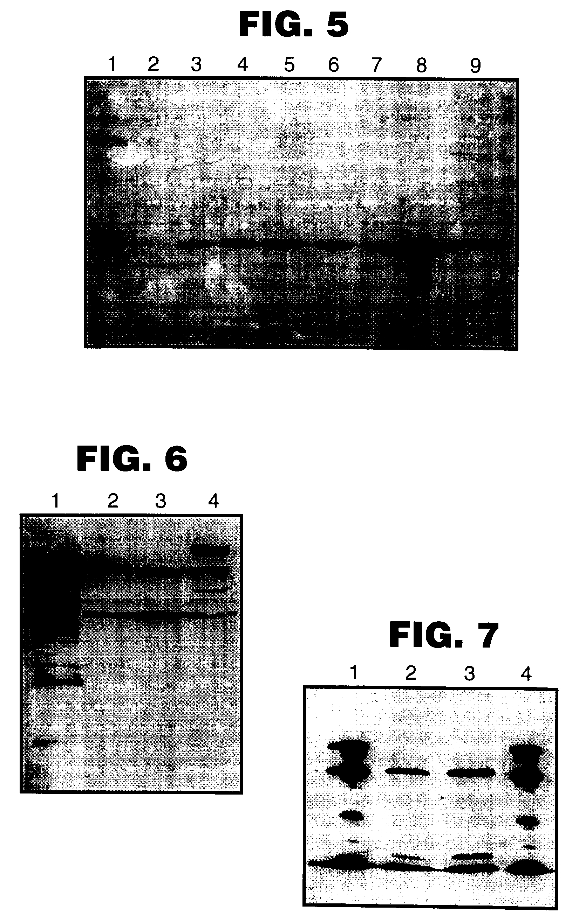

Based on the evidence that Nap is able to form decamer of 150 kDa when produced by *H.pylori,* we investigated whether the Nap produced by IEM101 is able to form the same kind of polymer. For this purpose, the recombinant IEMnirNAP strain was grown overnight at 37°C in aeration, the culture was centrifuged, the bacterial cells resuspended in PBS and sonicated. After sonication the sample was centrifuged and the supernatant, containing the Nap protein, was isolated and loaded on a Superose 12HR column. The elution volume of the aldolase, a protein with a molecular weight of 158kDa was used as standard for the gel filtration. The fractions were collected, loaded on a SDS gel and characterised by western blotting, using anti-NAP polyclonal rabbit antibodies. As shown in Figure 5, the fractions 24 through 28 corresponding to the elution volume of aldolase, contain the Nap protein, indicating that even in IEM101, Nap is able to form decamers of 150kDa, which are disassembled to monomers of 15kDa when loaded on the SDS gel.

### 2.5 CagA expression in IEM101.

The plasmid pGEMnir-CagA containing the gene coding for the CagA antigen (cytotoxicity-associated immunodominant antigen of *H. pylori)* was introduced in IEM101 by electroporation. The recombinant IEM101nircagA strain was grown in 15ml of LB medium containing 100µg/ml of ampicillin, for 16 hours at 37°C with aeration. Two samples of the culture each of 1ml were centrifuged, and the supernatant was stored at 4°C until the analysis has been performed. The bacterial pellet was resuspended in 500µl of PBS and sonicated. After sonication the sample was centrifuged, and the supernatant, containing the soluble fraction was isolated.

To isolate the periplasmic fraction, the bacterial pellet was resuspended in 500µl of 50mM Tris pH 8, 25% sucrose treated with 1 mg/ml of polimixin B and incubated for 30 minutes at room temperature. The supernatant, containing the periplasmic fraction was isolated by centrifugation. 30µl of culture supernatant, 30µl of the soluble fraction and 30µl of periplasm were then loaded on a SDS gel and analysed by western blotting using anti-CagA rabbit polyclonal antibodies.

CagA is produced in *H. pylori* as a 128 kDa protein. When the protein is expressed and purified from recombinant *E. coli* strains, only a small amount of CagA is present in the 128 kDa form, but most of it is proteolysed in the two fragments of 96 and 21kDa respectively (Figure 6, lane 1). In IEM101, CagA is present in its proteolysed form in the soluble fraction and in the periplasm (lanes 2 and 3), indicating that the protein is efficiently secreted into the periplasm of IEM101. Furthermore, the CagA is present also in the supernatant of IEM101 and interestingly, it is present mainly in the 128kDa form (Figure 6, lane 4). These data indicate that IEM101 is not only able to express detectable amount of CagA but even to secrete it into the supernatant.

### 2.6 Expression of LTK63 in IEM101.

The plasmid containing the gene coding for LTK63 under the control of nirB promoter was compared for protein production with the plasmid containing the gene coding for CTK63 under the control of the pCT promoter. These plasmids were introduced into IEM101 by electroporation, generating IEMLTK63 and IEMCTK63 respectively. The two strains were grown in 15 ml of LB medium containing 100 µg/ml of ampicillin, for 16 hours at 37°C with aeration. 1 ml of each culture was centrifuged and 30µl of supernatant was analysed for the presence of both LTK63 and CTK63 by western blot using a mixture of anti-LT and anti-CT polyclonal antibodies. As shown in Figure 7, IEM101 is able to secrete both mutated toxins into the supernatant. Furthermore, IEM101 was found to be able to process the A subunit of LTK63 into the A1 and A2 domains.

### Example 3: Attenuated cholerae toxin strains as carriers for homologous antigens.

In a subsequent experiment, the gene encoding CTK63 (CT containing a Ser63Lys mutation) was inserted into the chromosome of the IEM101 strain by homologous recombination, using the haemolysis gene as an insertional locus.

Because the level of expression of the protein may affect the immunological response to toxin and the stability of the strain both *in vitro* (in fermenters) and *in vivo,* we have analyzed the expression of the mutated CT gene either from its own promoter or from an inducible promoter such as NirB.

The level of CTK63 mutant protein expressed by the recombinant strains has been evaluated by Western blot analysis. Results confirm that both the CT and the NirB promoters are active in the IEM101 strain, and that the level of expression of the CTK63 is higher when the mutated gene is under the control of the NirB promoter, compared to the CT promoter. Moreover, the activity of the NirB promoter is induced by anaerobic conditions and reaches its maximum after 4.5 hours of induction.

### REFERENCES

Acheson *et al.,* (1996) Infect Immun 64 p355.

Barry E.M., O.G. Gomez-Duarte, S. N. Chatfield, R. Rappuoli, M. Pizza, G.A. Losonsky, J. Galen and M.M. Levine (1996) Infect Immun **64(10)** pp4172-4181.

Burnette, W.N., V.L. Mar, B.W. Plater, J.D. Schlotterbeck, M.D. McGinley, K.S. Stoney, M.F. Rohde and H.R. Kaslow (1991) Infect immun **59(11)** pp4266-4270.

Butterton *et al.,* (1995) Infect Immun **63**, p2689.

Chatfield, S.N., N. Fairweather, I. Charles, D. Pickard, M. Levine, D. Hone, M. Posada, R.A. Strugnell, G. Dougan (1992) Vaccine **10(1)** pp53-60

Domenighini M., M. Pizza, M.G. Jobling, R.K. Holmes and R. Rappuoli (1995) Mol microbiol **15(6)** ppl 165-1167.

Gmnaro M.L. *et al.,* (1982) Nucl Acids Res **12** p4883.

Guancai, D.C. *et al.,* (1989) Chin J. Epidemiol. **10 (suppl 10)** p205.

Fairweather, N.F, S.N. Chatfield, A.J. Makoff, R.A. Strugnell, J. Bester, D.J. Maskell and G. Dougan (1990) Infect Immun **58(5)** pp 1323-1326.

Favre *et al.,* (1996) Infect Immun **64** p576.

Galen, J.E., O.G. Gomez-Duarte, G.A. Losonsky, J.L. Halpern, C.S. Lauderbaugh, S. Kaintuck, M.K. Reymann and M.M. Levine (1997) Vaccine **15(7)** pp700-708.

Harford, S., C.W. Dykes, A.N. Hobden, M.J. Read and I.J. Halliday (1989) Eur J Biochem **183(2)**pp311-316.

Manual of prevention and treatment of cholera in China. Ministry of public health of China, 1987.

Mosely S.L. *et al.,* (1983) Infect Immun **39** p1167.

Newland J.W. *et al.,* (1985) Science **20**, p179.

Oi G.M. and S.Y. Gao (1984) Prevention and treatment of cholera in China. Ministry of public health of China, 1984. pp294-303.

Pearson G.D.N. and J.J. Mekalanos (1982) P.N.A.S. USA **79**, p2976.

Roberts, M., A. Bacon, R. Rappuoli, M. Pizza, I. Cropley, G. Douce, G. Dougan, M. Marinaro, J. McGhee and S. Chatfield (1995) Infect Immun **63(6)** pp2100-2108.

Sambrook *et al.,* Molecular cloning, A laboratory manual. (1989) 2nd edition. Cold Spring Harbor Laboratory Press.

Sixma, T.K., S.E. Pronk, K.H. Kalk,, E.S. Wartna, B.A. van Zanten, B. Witholt and W.G. Hol (1991) Nature **351(6325)** pp371-377.

Tsuji, T., T. Inouye, A. Mikayama, K. Okamoto, T. Honda and T. Miwatani (1990) J. Biol. Chem. **265(36)** pp22520-22525.

Viret *et al.,* (1996) Mol microbiol **19** p949.

Wang S.X. *et al.,* (1992) Chin J. Epidemiol. **13 (suppl 7)** p222.

## Claims

1. An attenuated *Vibrio cholerae* bacterium that expresses a heterologous antigen or derivative thereof, provided that the heterologous antigen is not derived from an enteropathogenic bacterium.

2. An attenuated bacterium of the IEM101 *V. cholerae* strain that expresses a heterologous or mutated homologous antigen or derivative thereof.

3. An attenuated bacterium according to claim 1 or 2, wherein the heterologous antigen is derived from a bacterial or viral pathogen.

4. An attenuated bacterium according to claim 3, wherein the heterologous antigen is derived from *B. pertussis* or *H. pylori.*

5. An attenuated bacterium according to claim 4, wherein the heterologous antigen is NAP (neutrophil activating protein).

6. An attenuated bacterium according to claim 4, wherein the toxin is CagA (cytotoxicity associated immunodominant antigen).

7. An attenuated bacterium according to claim 4, wherein the toxin is VacA (vacuolating cytotoxin A).

8. An attenuated bacterium according to claim 3, wherein the heterologous antigen is a toxin or derivative thereof.

9. An attenuated bacterium according to claim 5, wherein the toxin is fragment C of the tetanus toxin or the tracheal colonisation factor of *B. pertussis.*

10. An attenuated bacterium according to claim 4, wherein the toxin is the heat-labile enterotoxin (LT) of *E. coli,* optionally mutated at position 63 of the wild type sequence (LTK63).

11. An attenuated bacterium according to claim 2, wherein the mutated homologous antigen is the CT protein.

12. An attenuated bacterium according to claim 2, wherein the mutated homologous antigen is the CTK63 protein.

13. An immunogenic composition for use as a vaccine comprising attenuated *V. cholerae* bacteria according to any one of claims 1 to 12 in conjunction with a pharmaceutically-acceptable carrier.

14. A vaccine composition comprising attenuated *V*. *cholerae* bacteria according to any one of claims 1 to 12 in conjunction with a pharmaceutically-acceptable carrier.

15. A vaccine composition according to claim 14 further comprising an adjuvant.

16. A process for the formulation of a vaccine composition according to claim 14 comprising bringing attenuated bacteria according to any one of claims 1 to 12 into association with a pharmaceutically-acceptable carrier.

17. A process for the formulation of a vaccine composition according to claim 14 comprising bringing attenuated bacteria according to any one of claims 1 to 12 into association with an adjuvant.

18. An attenuated bacterium according to any one of claims 1 to 12 for use as a pharmaceutical.

19. An attenuated bacterium according to any one of claims 1 to 12 for use as an adjuvant.

20. Use of an attenuated bacterium according to any one of claims 1 to 12 in a vaccine composition.

21. A method for the prevention or treatment of disease in a subject, comprising administering to said subject an immunologically effective dose of an immunogenic composition according to claim 12.

22. A method for the prevention or treatment of disease in a subject, comprising administering to said subject a vaccine composition according to either of claims 14 or 15.
